# EUROPEAN PATENT SPECIFICATION

(11) **EP 3 480 246 B1**
(45) Date of publication and mention of the grant of the patent: **14.04.2021**
(21) Application number: 17819401.5
(22) Date of filing: 29.06.2017
(51) Int. Cl.: C08L 1/02, C08B 1/00, C08J 3/11, C07D 233/58

(54) **MIXTURES OF SALTS FOR DISSOLVING CELLULOSE**
SALZGEMISCHE ZUR CELLULOSEAUFLÖSUNG
MÉLANGES DE SELS POUR DISSOUDRE DE LA CELLULOSE

(30) Priority: 01.07.2016 ES 201630898
(43) Date of publication of application: 08.05.2019
(73) Proprietor: Universidade de Santiago de Compostela, 15782 Santiago de Compostela (ES); Adam Mickiewicz University Foundation, 61-612 Poznan (PL)
(72) Inventor: RODRÍGUEZ MARTÍNEZ, Héctor, 15883 Teo (ES); SOTO CAMPOS, Ana María, 15895 Milladoiro-Ames A Coruña (ES); STOLARSKA, Olga, 60-509 Poznan (PL); SMIGLAK, Marcin, 61-680 Poznan (PL); PAWLOWSKA, Anna, 59-814 Pobiedna (PL)
(74) Representative: AOMB Polska Sp. z.o.o.
(86) International application number: PCT/ES2017/070470
(87) International publication number: WO 2018/002403

(56) References cited:
- WO-A1-2016/094878
- WO-A2-2011/056924
- US-A1- 2014 027 938
- JINXING LONG ET AL: "One step catalytic conversion of cellulose to sustainable chemicals utilizing cooperative ionic liquid pairs", GREEN CHEMISTRY, vol. 13, no. 9, 1 January 2011 (2011-01-01), page 2334, XP055047432, ISSN: 1463-9262, DOI: 10.1039/c1gc15597k
- Bin Guo ET AL: "Electronic Supplementary Information One Step Catalytic Conversion of Cellulose to Sustainable Chemicals Utilizing Cooperative Ionic Liquid Pairs Jinxing Long a", , 1 January 2011 (2011-01-01), XP55596383, Retrieved from the Internet: URL:http://www.rsc.org/suppdata/gc/c1/c1gc 15597k/c1gc15597k.pdf [retrieved on 2019-06-13]
- XIA, S. ET AL.: 'Aqueous ionic liquids and deep eutectic solvents for cellulosic biomass pretreatment and saccharification' RSC ADVANCES vol. 4, 2014, pages 10586 - 10596, XP055382581
- STOLARSKA, O. ET AL.: 'Properties modification by eutectic formation in mixtures of ionic liquids' RSC ADVANCES vol. 5, 15 February 2015, pages 22178 - 22187, XP055450625

## Description

### Sector of the invention

The present invention refers to a composition to dissolve biomass. More particularly, refers to a composition of ionic liquids and a method to dissolve biomass, in particular cellulose, its manufacturing process and method of use for inter alia sheets or films production.

### State of the Art

Cellulose is the most abundant biopolymer in Nature, and a popular feedstock for the production of chemicals and materials of a biorenewable origin. Processes to obtain these products often require the dissolution and regeneration of cellulose. Cellulose is difficult to dissolve due to the strong network of hydrogen bonds between and within its polymeric chains, which prohibits solvation by conventional solvents. Since traditional solvents are not suited well for cellulose dissolution, more efficient dissolution procedures are required (Heinze, T; Koschella, A. "Solvents applied in the field of cellulose chemistry - a mini review", Polimeros: Ciencia e Tecnologia, vol. 15, n°2, p. 84-90. 2005); and thus, the dissolution and regeneration of cellulose from ionic liquids is attracting considerable attention.

Ionic liquids are salts with a relatively low melting temperature (typically a maximum mark of 100 °C is considered in their definition). Many ionic liquids are liquid at room temperature and well below, whereas others remain in a solid state even up to several dozens of degrees centigrade above room temperature. In general, ionic liquids present a set of properties that make them attractive for their use as fluids in different processes and applications; an example can be their wide liquid range (including typical operation temperature ranges) in combination with a practically negligible vapour pressure, since they are constituted of ions.

The patent application WO 2003029329 A2 discloses that some ionic liquids are able to dissolve cellulose. However, the dissolution process has to be carried out, inevitably, at a temperature above the melting point of the ionic liquid. Temperatures between 70 and 100°C are necessary to dissolve up to 7wt% of cellulose with respect to the weight of the ionic liquid. Only in one case, when the mixture was heated under microwave conditions, the solution of cellulose was up to 20wt%. However these conditions are not appropriate for industrial application since the process can easily lead to degradation of the ionic liquids and cellulose, or even explosions of sealed systems (Chem. Soc. Rev., 2012, 41, 1519-1537).

The patent application WO 2011056924 A2 discloses statistical mixtures of ionic liquids to dissolve cellulose. These statistical mixtures are mixtures comprising different cations and/or anions, which are prepared by a process comprising the reaction of mixtures of ionic liquid precursors in one-pot. Those mixtures can dissolve up to 35wt% of cellulose when the mixture is heated at 100°C under microwave conditions, again conditions which are not safe neither scalable for industrial process.

Thus, new compositions to dissolve cellulose in higher amounts and under milder and scalable conditions are still required.

### Description of the invention

The authors of the present invention have found a mixture of ionic liquids that is able to dissolve cellulose. That mixture dissolves cellulose more effectively than other known compositions. Furthermore, the method of the invention, which employs this mixture, is carried out under mild conditions. Besides, the method avoids microwaves and other dangerous steps. Even more, the method of the invention is scalable.

The mixture of the invention preserves some of the intrinsic ionic liquid features, such as negligible vapour pressure. It is preferred that the mixture of the invention has a eutectic behaviour.

Thus, the authors of the present invention have found that some ionic liquids can be combined to yield eutectic systems, and these ionic liquid mixtures can be used to dissolve cellulose at temperatures below the melting temperatures of the pure ionic liquids; i.e., at temperatures at which it would not be possible to carry out the dissolution of cellulose in the pure ionic liquids. Preferred ionic liquids in the mixture are imidazolium ionic liquids having chloride or acetate as anions.

Thus, an aspect of the invention refers to a composition comprising a mixture of two or more imidazolium ionic liquids and between 0.1 and 50%wt of cellulose where the mixed ionic liquids configure a eutectic system and their anions are selected from chloride and acetate.

A particular embodiment refers to the composition disclosed above, wherein the imidazolium ionic liquids are 1-(C2-C6)alkyl-3-(C2-C6)alkyl-imidazolium.

A particular embodiment refers to the composition disclosed above, wherein the ionic liquids are selected from 1-ethyl-3-methylimidazolium chloride, 1-ethyl-3-methylimidazolium acetate and 1-butyl-3-methylimidazolium chloride.

Other particular embodiment refers to the composition disclosed above, wherein two of those ionic liquids are in a relation between 1:10 to 10:1; preferably wherein two of those ionic liquids are in a relation between 1:5 to 5:1; more preferably wherein two of those ionic liquids are in a relation between 1:3 to 3:1.

Other particular embodiment refers to the composition disclosed above, comprising 1-ethyl-3-methylimidazolium chloride and at least one more ionic liquid selected from 1-ethyl-3-methylimidazolium acetate and 1-butyl-3-methylimidazolium chloride in a relation between 1:5 to 5:1.

Other particular embodiment refers to the composition disclosed above, comprising 1-ethyl-3-methylimidazolium chloride and at least one more ionic liquid selected from 1-ethyl-3-methylimidazolium acetate and 1-butyl-3-methylimidazolium chloride in a relation between 1:3 to 3:1.

Other particular embodiment refers to the composition disclosed above, selected from:
i) 1-ethyl-3-methylimidazolium chloride and 1-ethyl-3-methylimidazolium acetate in a 3:7 molar ratio,
ii) 1-ethyl-3-methylimidazolium chloride and 1-butyl-3-methylimidazolium chloride in a 1:1 molar ratio, and
iii)) 1-ethyl-3-methylimidazolium chloride in a molar ratio from 1:5 to 5:1 with mixture of 1-ethyl-3-methylimidazolium acetate and 1-butyl-3-methylimidazolium chloride in any molar ratio.

Other particular embodiment refers to the composition disclosed above, further comprising a viscosity-reducing agent, preferably dimethyl sulfoxide, in an amount from 0.1 to 70%wt. Other particular embodiment refers to the composition disclosed above, wherein the cellulose is between 0,1 and 40%wt.

In a preferred embodiment, the composition of the invention is a liquid composition.

In a preferred embodiment, the mixture of ionic liquids displays a eutectic behaviour. In a particular embodiment, the mixture of ionic liquids is a mixture of eutectic composition.

When the mixture of ionic liquids described in the present invention is used to dissolve cellulose, the yield of the dissolution process can be up to 10 times the yield obtained when one of those ionic liquids is used alone.

The best yield for cellulose dissolution is obtained when the composition comprises 1-ethyl-3-methylimidazolium chloride and 1-ethyl-3-methylimidazolium acetate in a 3:7 molar ratio. A more preferred embodiment of the invention refers to the composition of the invention comprising 1-ethyl-3-methylimidazolium chloride and 1-ethyl-3-methylimidazolium acetate in a 3:7 molar ratio.

Other aspect of the invention refers to a method of obtaining the composition as disclosed above, comprising:
a) mixing cellulose in an amount of from 0.1 to 50wt% with a mixture of two or more imidazolium ionic liquids, wherein the ionic liquids mixed configure a eutectic system and their anions are selected from chloride and acetate, and
b) heating the mixture obtained in step (a) at a temperature below 105°C.

A preferred embodiment refers to the method of the invention as previously described, wherein the imidazolium ionic liquids are 1-(C2-C6)alkyl-3-(C2-C6)alkyl-imidazolium. More preferably, the imidazolium ionic liquids are selected from 1-ethyl-3-methylimidazolium chloride, 1-ethyl-3-methylimidazolium acetate, and 1-butyl-3-methylimidazolium chloride. Preferably, two of those ionic liquids are in a relation between 1:10 to 10:1; more preferably two of those ionic liquids are in a relation between 1:5 to 5:1; even more preferably, they are in a relation between 1:3 to 3:1.

Another preferred embodiment refers to the method of the invention as previously described, wherein the imidazolium ionic liquids comprise 1-ethyl-3-methylimidazolium chloride and at least one more ionic liquid selected from 1-ethyl-3-methylimidazolium acetate and 1-butyl-3-methylimidazolium chloride in a relation between 1:5 to 5:1, preferably in a relation between 1:3 to 3:1.

A preferred embodiment refers to the method of the invention as previously described, wherein the mixture of imidazolium ionic liquids is selected from
i) 1-ethyl-3-methylimidazolium chloride and 1-ethyl-3-methylimidazolium acetate in a 3:7 molar ratio,
ii) 1-ethyl-3-methylimidazolium chloride and 1-butyl-3-methylimidazolium chloride in a 1:1 molar ratio, and
iii) 1-ethyl-3-methylimidazolium chloride in a molar ratio from 1:5 to 5:1 with a mixture of 1-ethyl-3-methylimidazolium acetate and 1-butyl-3-methylimidazolium chloride in any molar ratio.

The mixture of ionic liquids disclosed in the present invention is so effective pretreating or dissolving biomass, in particular cellulose, that the method of the invention can be performed in mild conditions. Thus, in a particular embodiment, the temperature of the step (b) is between 50°C and 105°C. In a more preferred embodiment, the temperature of the step (b) is between 70°C and 100°C.

A more preferred embodiment refers to the method of the invention, comprising:
a) mixing the cellulose with 1-ethyl-3-methylimidazolium chloride and 1-ethyl-3-methylimidazolium acetate in a 3:7 molar ratio, and
b) heating the mixture obtained in step (a) at a temperature below 80°C, preferably at a temperature between 60°C and 80°C, more preferably at 70°C.

Optionally, a viscosity-reducing agent can be further added to step (a), as for example dimethylsulfoxide, in an amount from 0.1 to 70wt%.

In another aspect, the invention refers to a composition obtainable by the method described above.

In another aspect, the invention refers to the use of the composition of the invention as previously disclosed to prepare cellulosic fibers, beads, sheets and other elements.

In another aspect, the invention refers to the use of the composition of the invention as previously disclosed to act as non-derivatising solvent for the chemical modification of cellulose in solution.

By using the presented invention the following effects and technical usage have been achieved:
- imidazolium salt mixture, which efficiently dissolve the biomass, in particular cellulose, have been obtained,
- the process of biomass dissolution requires relatively mild conditions, it does not require the use of microwaves and other hazardous process conditions, making it suitable for use on an industrial scale,
- preservation of characteristic properties of ionic liquids, such as negligible vapor pressure, while the system shows a eutectic behavior,
- a mixture of ionic liquids that remains in liquid state at a specified temperature range, usually lower than the melting point of pure ionic liquids.
- the melting point of the mixture of eutectic composition is lower than the melting point of the mixtures of the same compounds in any composition other than the eutectic composition,
- the resulting mixtures can dissolve cellulose at a lower temperature than the melting point of pure parent salts, thus in the temperature which normally would not be possible to carry out this process in a pure ionic liquid,
- Possibility to use the composite to produce e.g. sheets, foils.

### Description of the figures

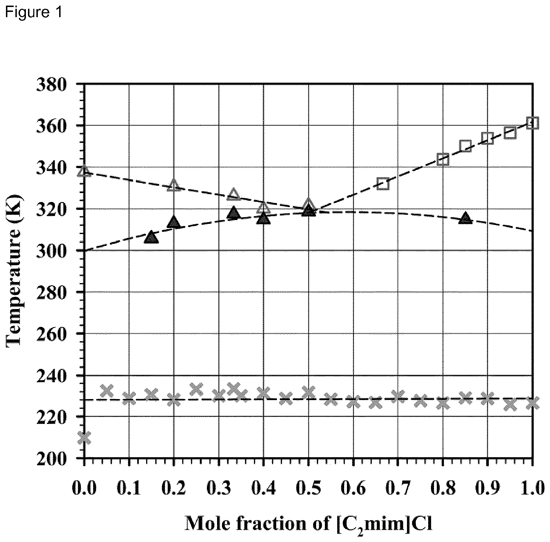

Figure 1. Temperature-composition phase diagram for the system [C₄mim]Cl + [C₂mim]Cl. Legend - Δ- melting point of the excess component [C₄mim]Cl in the mixture; -□- melting point of the excess component [C₂mim]Cl in the mixture; -▲- eutectic mixture thermal transition; -X- glass transition observed below the eutectic point transition.

Next, for a greater understanding of the characteristics and advantages of the present invention, reference is made to a number of explanatory examples which complete the previous description, without the invention being limited in any way to them.

### Materials:

The ionic liquids 1-ethyl-3-methylimidazolium chloride, 1-ethyl-3-methylimidazolium acetate, and 1-butyl-3-methylimidazolium chloride were purchased from commercial vendors with a quoted purity >95% for 1-ethyl-3-methylimidazolium acetate, and >98% for 1-ethyl-3-methylimidazolium chloride and 1-butyl-3-methylimidazolium chloride. All salts were dried under vacuum for 72 h at 90 °C, achieving a final water content <0.9% determined by Karl Fischer titration using a Metrohm 831 KF coulometer.

Microcrystalline cellulose (Cellulose no. 435236) was purchased from a commercial vendor, and used as received.

### Example 1. Characterisation of the eutectic behaviour of an exemplary ionic liquid mixture system of the invention.

Several mixtures of the ionic liquid 1-ethyl-3-methylimidazolium chloride ([C₂mim]Cl), with a melting point of 88 °C, and the ionic liquid 1-butyl-3-methylimidazolium chloride ([C₄mim]Cl), with a melting point of 65 °C, were prepared by dissolving each ionic liquid separately in methanol, combining both methanol solutions in the desired proportion, and evaporating completely the methanol. Mixtures were prepared so that their compositions ranged from pure [C₂mim]Cl to pure [C₄mim]Cl, evenly distributed over the entire composition range at intervals of 5 mol%. A sample of each mixture was subjected to analysis by differential scanning calorimetry (DSC). By plotting the signals of the corresponding thermograms in a temperature-composition phase diagram, the existence of a eutectic behaviour could be corroborated. By extrapolation of the trend of the signals of the excess components in such diagram (Fig 1), a melting temperature of 46 °C and a composition of 0.51 in mole fraction of [C₂mim]Cl and 0.49 in molar fraction of [C₄mim]Cl were identified for the eutectic point of this particular eutectic mixture.

### Example 2. Preparation of an exemplary ionic liquid mixture of the invention.

The ionic liquid 1-ethyl-3-methylimidazolium chloride ([C₂mim]Cl) has a melting temperature of 88 °C, and therefore it is a solid at room temperature (for instance 22 °C). The ionic liquid 1-ethyl-3-methylimidazolium acetate ([C₂mim][OAc]) is a liquid at room temperature and also at much lower temperatures, with no melting temperature above -73 °C. These two ionic liquids were mixed in a vial in a 3:7 molar ratio (previously determined as the eutectic composition for this system), and the mixture was heated and stirred for 10 minutes at 100 °C. A homogeneous liquid was obtained, which remained stable in that condition when cooled down to room temperature. No melting temperature could be detected by DSC for the obtained mixture above -73 °C.

### Example 3. Cellulose dissolution in the mixture of 1-ethyl-3-methylimidazolium chloride and 1-ethyl-3-methylimidazolium acetate at 75 °C.

5 g of a 3:7 molar ratio mixture of [C₂mim]Cl and [C₂mim][OAc] was placed in a jacketed glass vessel equipped with an overhead mechanical stirrer. The mixture was heated to 75 °C, and 0.5 g of microcrystalline cellulose was added. The content of the thermostated vessel was stirred until complete dissolution of the cellulose (visually clear solution). 0.25 g was subsequently added, and then the content was newly stirred until complete dissolution of the cellulose. This action was repeated several times. After addition of a total amount of 1.50 g of cellulose (corresponding to 30 g of cellulose per 100 g of ionic liquid mixture), a clear solution was still obtained. No further additions of cellulose were made, because at this point the solution had turned excessively viscous.

### Example 4. Cellulose dissolution in the mixture of 1-ethyl-3-methylimidazolium chloride and 1-ethyl-3-methylimidazolium acetate at 100 °C.

5 g of a 3:7 molar ratio mixture of [C₂mim]Cl and [C₂mim][OAc] was placed in a jacketed glass vessel equipped with an overhead mechanical stirrer. The mixture was heated to 100 °C, and 0.5 g of microcrystalline cellulose was added. The content of the thermostated vessel was stirred until complete dissolution of the cellulose (visually clear solution). 0.25 g was subsequently added, and then the content was newly stirred until complete dissolution of the cellulose. This action was repeated several times. After addition of a total amount of 2.00 g of cellulose (corresponding to 40 g of cellulose per 100 g of ionic liquid mixture), a clear solution was still obtained.

### Example 5. Cellulose dissolution in the mixture of 1-ethyl-3-methylimidazolium chloride and 1-butyl-3-methylimidazolium chloride.

5 g of a mixture of the ionic liquid [C₂mim]Cl and [C₄mim]Cl at a molar ratio 49:51 (eutectic composition) was prepared and placed in a jacketed glass vessel equipped with a mechanical stirrer, and was heated to 50 °C, which is below the melting point of both [C₂mim]Cl and [C₄mim]Cl. Microcrystalline cellulose (0.5 g) was added. The solution (with a concentration of 10 g of cellulose per 100 g of ionic liquid mixture) was stirred mechanically until the complete dissolution of the cellulose, obtaining an optically clear and viscous solution.

### Example 6. Influence of dimethylsulfoxide (DMSO) addition on the process of cellulose dissolution in ionic liquid mixtures.

4.25 g of a 3:7 molar ratio mixture of [C₂mim]Cl and [C₂mim][OAc] and 0.75 g DMSO was placed in a jacketed glass vessel equipped with an overhead mechanical stirrer. The mixture was heated to 75 °C, and 0.5 g of microcrystalline cellulose was added. The content of the thermostated vessel was stirred until complete dissolution of the cellulose (visually clear solution). 0.25 g of microcrystalline cellulose was subsequently added, and then the content was stirred again until complete dissolution of the cellulose. Further additions of microcrystalline cellulose and subsequent dissolution were carried out. After addition of a total amount of 1.75 g of cellulose (corresponding to 35 g of cellulose per 100 g of ionic liquid mixture), a clear solution was still obtained. No further additions of cellulose were made, because at this point the solution turned into gel.

### Example 7.

A mixture of imidazolium salts: 1-ethyl-3-methylimidazolium chloride [C₂mim]Cl in a molar ratio of from 1:5 to 5:1 with a mixture of 1-ethyl-3-methylimidazolium acetate [C₂mim][OAc] and 1-butyl-3-methylimidazolium chloride [C₄mim]Cl in any molar ratio, prepared in an analogous manner as in Example 1, was placed in a jacketed glass vessel equipped with an overhead mechanical stirrer. The mixture was heated to 75°C, and microcrystalline cellulose in an amount greater than 30 g of cellulose to 100 g of the used mixture of imidazolium salts was added. During the addition of cellulose, the content of the reactor was thermostated and stirred until the complete dissolution of the cellulose, obtaining an optically clear and viscous solution.

### Example 8. Inability of the eutectic mixture of 1-ethyl-3-methylimidazolium nitrate and 1-ethyl-3-methylimidazolium hexafluorophosphate to dissolve cellulose

As an example of ionic liquid eutectic mixture not able to dissolve cellulose, the eutectic mixture of 1-ethyl-3-methylimidazolium nitrate ([C₂mim][NO₃]) and 1-ethyl-3-methylimidazolium hexafluorophosphate ([C₂mim][PF₆] was used. The ionic liquids were mixed in a 60:40 molar ratio to form a eutectic mixture (with a melting point of 19 °C). 5 g of the prepared mixture was placed in a jacketed glass vessel equipped with a mechanical stirrer, and was heated to 75 °C. Microcrystalline cellulose (0.5 g) was added, and the content of the vessel mechanically stirred. After 48 h at those conditions, cellulose remained suspended in the ionic liquid mixture, forming a cloudy paste, with no signs of dissolution.

### Example of use

The composite prepared according to Example 5, is reprocessed in a known manner, to obtain film or foil. It yields the product with properties comparable to the products obtained using currently known methods.

## Claims

1. A composition comprising a mixture of two or more imidazolium ionic liquids and between 0.1 and 50%wt of cellulose, wherein the mixed ionic liquids configure a eutectic system and their anions are selected from chloride and acetate.

2. The composition according to claim 1, wherein the imidazolium ionic liquids are 1-(C2-C6)alkyl-3-(C2-C6)alkyl-imidazolium.

3. The composition according to claims 1- 2, wherein the ionic liquids are selected from 1-ethyl-3-methylimidazolium chloride, 1-ethyl-3-methylimidazolium acetate, and 1-butyl-3-methylimidazolium chloride.

4. The composition according to any of previous claims, wherein two of those ionic liquids are in a relation between 1:10 to 10:1, preferably between 1:5 to 5:1, more preferably between 1:3 to 3:1.

5. The composition according to any of previous claims, comprising 1-ethyl-3-methylimidazolium chloride and at least one more ionic liquid selected from 1-ethyl-3-methylimidazolium acetate and 1-butyl-3-methylimidazolium chloride in a relation between 1:5 to 5:1, preferably between 1:3 to 3:1 .

6. The composition according to any of previous claims, selected from:
i) 1-ethyl-3-methylimidazolium chloride and 1-ethyl-3-methylimidazolium acetate in a 3:7 molar ratio,
ii) 1-ethyl-3-methylimidazolium chloride and 1-butyl-3-methylimidazolium chloride in a 1:1 molar ratio,
iii)) 1-ethyl-3-methylimidazolium chloride in a molar ratio from 1:5 to 5:1 with mixture of 1-ethyl-3-methylimidazolium acetate and 1-butyl-3-methylimidazolium chloride in any molar ratio.

7. The composition according to any of previous claims, further comprising a viscosity-reducing agent, preferably dimethyl sulfoxide, in an amount from 0.1 to 70%wt.

8. A method of obtaining the composition as described in claims 1-7, comprising:
a) mixing cellulose in an amount of from 0.1 to 50wt% with a mixture of two or more imidazolium ionic liquids, wherein the mixed ionic liquids configure a eutectic system and their anions are selected from chloride and acetate, and
b) heating the mixture obtained in step (a) at a temperature below 105°C.

9. The method according to claim 8, wherein the temperature in the step (b) is between 50°C and 105°C, preferably between 70°C and 100°C .

10. The method according to any of claims 8-9, comprising:
a) mixing the cellulose with 1-ethyl-3-methylimidazolium chloride and 1-ethyl-3-methylimidazolium acetate in a 3:7 molar ratio, and
b) heating the mixture obtained in step (a) at a temperature below 80°C.

11. The method according to any of claims 8-10, wherein a viscosity-reducing agent is further added to step (a) in an amount from 0.1 to 70wt%.

12. Use of the composition according to any of claims 1-7, to prepare cellulose fibers, beads, sheets, and other elements.

13. Use of the composition according to any of claims 1-7, as non-derivatizing solvent for the chemical modification of cellulose in solution.

## Patentansprüche

1. Eine Zusammensetzung, umfassend eine Mischung aus zwei oder mehr ionischen Imidazoliumflüssigkeiten und zwischen 0,1 und 50 Gew .-% Cellulose, wobei die gemischten ionischen Flüssigkeiten ein eutektisches System bilden und ihre Anionen aus Chlorid und Acetat ausgewählt sind.

2. Die Zusammensetzung nach Anspruch 1, wobei die ionischen Imidazoliumflüssigkeiten 1-(C2-C6)-Alkyl-3-(C2-C6)-Alkyl-imidazolium sind.

3. Die Zusammensetzung nach Anspruch 1-2, wobei die ionischen Flüssigkeiten ausgewählt sind aus 1-Ethyl-3-methylimidazoliumchlorid, 1-Ethyl-3-methylimidazoliumacetat und 1-Butyl-3-methylimidazoliumchlorid.

4. Die Zusammensetzung nach einem der vorhergehenden Ansprüche, wobei zwei dieser ionischen Flüssigkeiten in einem Verhältnis zwischen 1:10 bis 10:1, vorzugsweise zwischen 1:5 bis 5:1, noch bevorzugter zwischen 1:3 bis 3:1 stehen.

5. Die Zusammensetzung nach einem der vorhergehenden Ansprüche, umfassend 1-Ethyl-3-methylimidazoliumchlorid und mindestens eine weitere ionische Flüssigkeit, ausgewählt aus 1-Ethyl-3-methylimidazoliumacetat und 1-Butyl-3-methylimidazoliumchlorid in einem Verhältnis zwischen 1:5 bis 5:1, vorzugsweise zwischen 1:3 bis 3:1.

6. Die Zusammensetzung nach einem der vorhergehenden Ansprüche, ausgewählt aus:
i) 1-Ethyl-3-methylimidazoliumchlorid und 1-Ethyl-3-methylimidazoliumacetat in einem molaren Verhältnis von 3:7,
ii) 1-Ethyl-3-methylimidazoliumchlorid und 1-Butyl-3-methylimidazoliumchlorid in einem molaren Verhältnis von 1:1,
iii)) 1-Ethyl-3-methylimidazoliumchlorid in einem molaren Verhältnis von 1:5 bis 5:1 mit einer Mischung aus 1-Ethyl-3-methylimidazoliumacetat und 1-Butyl-3-methylimidazoliumchlorid in einem beliebigen molaren Verhältnis.

7. Die Zusammensetzung nach einem der vorhergehenden Ansprüche, ferner umfassend ein viskositätsverringerndes Mittel, vorzugsweise Dimethylsulfoxid, in einer Menge von 0,1 bis 70 Gew.-%.

8. Ein Verfahren zum Erhalten der Zusammensetzung, wie in den Ansprüchen 1-7 beschrieben, wobei das Verfahren umfasst:
(a) Mischen von Cellulose in einer Menge von 0,1 bis 50 Gew .-% mit einer Mischung von zwei oder mehr ionischen Imidazoliumflüssigkeiten, wobei die gemischten ionischen Flüssigkeiten ein eutektisches System bilden und ihre Anionen aus Chlorid und Acetat ausgewählt sind, und
(b) Erhitzen der in Schritt (a) erhaltenen Mischung auf eine Temperatur unter 105°C.

9. Das Verfahren nach Anspruch 8, wobei die Temperatur im Schritt (b) zwischen 50°C und 105°C, vorzugsweise zwischen 70°C und 100°C liegt

10. Das Verfahren nach einem der Ansprüche 8 bis 9, umfassend:
(a) Mischen der Cellulose mit 1-Ethyl-3-methylimidazoliumchlorid und 1-Ethyl-3-methylimidazoliumacetat in einem molaren Verhältnis von 3:7, und
(b) Erhitzen der in Schritt (a) erhaltenen Mischung auf eine Temperatur unter 80°C.

11. Das Verfahren nach einem der Ansprüche 8-10, wobei ein viskositätsverringerndes Mittel weiter zu Schritt (a) in einer Menge von 0,1 bis 70 Gew.-% zugegeben wird.

12. Verwendung der Zusammensetzung nach einem der Ansprüche 1 bis 7 zur Herstellung von Cellulosefasern, -perlen, -folien und anderen Elementen.

13. Verwendung der Zusammensetzung nach einem der Ansprüche 1 bis 7 als nichtderivatisierendes Lösungsmittel zur chemischen Modifizierung von Cellulose in Lösung.

## Revendications

1. Une composition comprenant un mélange de deux ou plusieurs liquides ioniques d'imidazolium et entre 0,1 et 50% en poids de cellulose, dans laquelle les liquides ioniques mélangés forment un système eutectique et leurs anions sont choisis parmi le chlorure et l'acétate.

2. La composition selon la revendication 1, dans laquelle les liquides ioniques d'imidazolium sont des 1-(C2-C6)alkyl-3-(C2-C6)alkyl-imidazolium.

3. La composition selon les revendications 1 et 2, dans laquelle les liquides ioniques sont choisis parmi le chlorure de 1-éthyl-3-méthylimidazolium, l'acétate de 1-éthyl-3-méthylimidazolium et le chlorure de 1-butyl-3-méthylimidazolium.

4. La composition selon l'une quelconque des revendications précédentes, dans laquelle deux de ces liquides ioniques sont dans un rapport compris entre 1:10 et 10:1, de préférence entre 1:5 et 5:1, plus préférablement entre 1:3 et 3:1.

5. La composition selon l'une quelconque des revendications précédentes, comprenant du chlorure de 1-éthyl-3-méthylimidazolium et au moins un liquide ionique supplémentaire choisi parmi l'acétate de 1-éthyl-3-méthylimidazolium et le chlorure de 1-butyl-3-méthylimidazolium dans un rapport compris entre 1:5 et 5:1, de préférence entre 1:3 et 3:1.

6. La composition selon l'une quelconque des revendications précédentes, choisie parmi:
i) chlorure de 1-éthyl-3-méthylimidazolium et acétate de 1-éthyl-3-méthylimidazolium dans un rapport molaire de 3: 7,
ii) chlorure de 1-éthyl-3-méthylimidazolium et chlorure de 1-butyl-3-méthylimidazolium dans un rapport molaire de 1:1,
iii) chlorure de 1-éthyl-3-méthylimidazolium dans un rapport molaire de 1:5 à 5:1 avec un mélange d'acétate de 1-éthyl-3-méthylimidazolium et de chlorure de 1-butyl-3-méthylimidazolium dans n'importe quel rapport molaire.

7. La composition selon l'une quelconque des revendications précédentes, comprenant en outre un agent réducteur de viscosité, de préférence du diméthylsulfoxyde, en une quantité de 0,1 à 70% en poids.

8. Une méthode pour obtenir la composition décrite dans les revendications 1 à 7, comprenant :
(a) le mélange de cellulose en une quantité de 0,1 à 50 % en poids avec un mélange de deux ou plusieurs liquides ioniques d'imidazolium, dans lequel les liquides ioniques mélangés forment un système eutectique et leurs anions sont choisis parmi le chlorure et l'acétate, et
(b) le chauffage du mélange obtenu à l'étape a) à une température inférieure à 105°C.

9. La méthode selon la revendication 8, dans laquelle la température dans l'étape (b) est comprise entre 50°C et 105°C, de préférence entre 70°C et 100°C .

10. La méthode selon l'une quelconque des revendications 8-9, comprenant:
(a) le mélange de la cellulose avec du chlorure de 1-éthyl-3-méthylimidazolium et de l'acétate de 1-éthyl-3-méthylimidazolium dans un rapport molaire de 3:7, et
(b) le chauffage du mélange obtenu à l'étape a) à une température inférieure à 80°C.

11. La méthode selon l'une quelconque des revendications 8 à 10, dans laquelle un agent réducteur de viscosité est en outre ajouté à l'étape (a) en une quantité de 0,1 à 70 % en poids.

12. Utilisation de la composition selon l'une quelconque des revendications 1 à 7, pour préparer des fibres de cellulose, des perles, des feuilles et d'autres éléments.

13. Utilisation de la composition selon l'une quelconque des revendications 1 à 7, comme solvant non dérivatif pour la modification chimique de la cellulose en solution.
